(19) Europäisches Patentamt
European Patent Office
Office européen des brevets

(11) EP 2 618 927 B1

(12) FASCICULE DE BREVET EUROPEEN

(45) Date de publication et mention de la délivrance du brevet:
**10.05.2017 Bulletin 2017/19**

(21) Numéro de dépôt: **11773742.9**

(22) Date de dépôt: **22.09.2011**

(51) Int Cl.:
***B01J 13/18*** *(2006.01)* ***C11D 3/50*** *(2006.01)*

(86) Numéro de dépôt international:
**PCT/FR2011/052192**

(87) Numéro de publication internationale:
**WO 2012/038666 (29.03.2012 Gazette 2012/13)**

# (54) PROCÉDÉ DE FABRICATION DE MICROCAPSULES POLYSILOXANE FONCTIONNALISÉES ET PEU POREUSES.

VERFAHREN ZUR HERSTELLUNG VON FUNKTIONALISIERTEN UND NICHT SEHR PORÖSEN POLYSILOXAN-MIKROKAPSELN

PROCESS FOR MANUFACTURING POLYSILOXANE MICROCAPSULES THAT ARE FUNCTIONALIZED AND ARE NOT VERY POROUS.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.09.2010 FR 1057708**

(43) Date de publication de la demande:
**31.07.2013 Bulletin 2013/31**

(73) Titulaire: **Université de Tours Francois Rabelais**
**37000 Tours (FR)**

(72) Inventeurs:
- **VIAUD-MASSUARD, Marie-Claude**
  **F-37100 Tours (FR)**
- **MONFRAY, Jérémy**
  **F-69003 Lyon (FR)**
- **ROBERT, Peggy**
  **F-41000 Blois (FR)**
- **RAYNAUD, Elodie**
  **B-7000 Mons (BE)**
- **BOUAZZA, Vincent**
  **F-11100 Narbonne (FR)**

(74) Mandataire: **Pontet Allano & Associes**
**Parc Les Algorithmes, Bâtiment Platon**
**CS 70003 Saint-Aubin**
**91192 Gif-sur-Yvette Cedex (FR)**

(56) Documents cités:
**FR-A1- 2 937 248**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 618 927 B1

## Description

La présente invention concerne un procédé d'encapsulation de produits pouvant présenter des propriétés lipophiles ou hydrophiles dans une membrane polysiloxane particulièrement étanche. Une méthode d'évaluation de l'étanchéité de telles capsules est aussi décrite. Des microcapsules sont utilisées dans beaucoup de domaines pour contenir et délivrer de façon dosée voire programmée des produits, par exemple des produits actifs voire des médicaments, dans de nombreux cas. De façon courante, il s'agit par exemple d'inclure un produit actif au sein d'un cosmétique. De plus en plus, des applications sont proposées pour inclure des microcapsules de produits actifs dans tout type d'objet, par exemple dans des objets textiles parfois appelés alors « texticaments », pour délivrer ces produits actifs au cours de la vie et de l'utilisation de tels objets.

La plupart du temps, la membrane des microcapsules est constituée de matériaux organiques comme des polymères ou des huiles. Cependant, la plupart des membranes polymères ont de mauvaises propriétés chimiques, une faible résistance mécanique et à la température. Il existe cependant quelques alternatives intéressantes telles que les résines mélamine formol mais la législation, de plus en plus drastique les concernant, empêchera à terme leur utilisation.

L'utilisation d'une membrane à base de polysiloxane permet de résoudre un certain nombre de ces inconvénients, comme proposé par exemple par le brevet US 6 855 335 qui décrit la fabrication de microcapsules par hydrolyse basique de TEOS en présence d'APS. Ces techniques présentent cependant des temps de process relativement long, par exemple de l'ordre de 15 à 24 heures, et des problèmes de fiabilité au niveau des structures obtenues.

Le brevet EP 2 080 552 propose une méthode d'encapsulation de produit lipophile dans une membrane polysiloxane à base de tetraethoxysilane (TEOS) et de methyltriethoxysilane (MTES). Cette méthode présente de grands avantages en matière d'innocuité des composants, ainsi qu'une bonne régularité des microcapsules et des temps de process améliorés, de l'ordre de 4 à 5 heures.

Toutefois, les microcapsules obtenues sont assez poreuses, ce qui rend délicate et peu fiable l'encapsulation de produits volatils, souvent des produits lipophiles, par exemple des parfums.

Le document FR 2 937 248 divulgue un procédé d'encapsulation d'un principe actif dans une enveloppe polymère formée à partir d'un composé de type silsesquioxane, qui utilise un ou plusieurs acides forts pour l'hydrolyse, comme par exemple de l'acide fluorhydrique. Or de tels acides sont très gênants pour de nombreuses applications, par exemple pour des produits qui doivent être en contact avec un utilisateur.

Un but de l'invention est de pallier les inconvénients de l'art antérieur.

Plus particulièrement, un but de l'invention est d'améliorer l'étanchéité des capsules tout en conservant de bonnes performances et les bonnes qualités de régularité des structures obtenues et d'innocuité telles qu'obtenues par le procédé décrit dans le brevet EP 2 080 552.

Par ailleurs, il peut être utile d'associer les microcapsules obtenues avec d'autres composés auxquels elles sont mélangées ou combinées.

Un autre but de l'invention est aussi de fournir des capsules comprenant en surface des groupements permettant une fonctionnalisation de ces capsules.

### Exposé de l'invention

L'invention propose pour cela un procédé d'encapsulation d'un produit lipophile dans une membrane polysiloxane, par exemple des huiles, des beurres, des parfums. Selon l'invention, le procédé d'encapsulation comprend les étapes suivantes :

a)- formation de gouttelettes par une émulsion entre une phase huileuse contenant le produit à encapsuler et une phase aqueuse acide chauffée aux environs de 50°C et en présence d'agents tensio-actifs ;
b)- ajout et hydrolyse d'au moins un silane pour obtenir un silanol ;
c)- augmentation du pH pour obtenir un début de condensation du silanol pour former une première membrane autour des gouttelettes du produit à encapsuler ;
d)- baisse du pH ;
e)- augmentation du pH pour obtenir une nouvelle ou meilleure condensation du ou des silanol(s) autour des gouttelettes du produit à encapsuler.

Des capsules contenant des produits présentant des propriétés hydrophiles peuvent aussi être obtenues selon l'invention.

Selon une particularité de l'invention, l'étape e) comprend en outre un ajout d'au moins un silane, par exemple avant l'augmentation du pH, possiblement un silane préhydrolysé.

De façon optionnelle, le silane ajouté lors de l'étape e) peut en outre être, ou comprendre, un silane différent

du ou des silanes ajoutés lors de l'étape b), voire un silane qui ne se liera pas avec lui ou avec eux. Il se forme ainsi autour des gouttelettes du produit à encapsuler une deuxième membrane lors de la nouvelle condensation de silanol au cours de l'étape e).

L'invention permet ainsi d'obtenir une membrane à double couche, ou double membrane, améliorant encore l'étanchéité, en particulier pour contenir des substances volatiles.

Le procédé selon l'invention représente une durée du même ordre de grandeur que l'antérieur décrit par le document EP 2 080 552, voire un peu plus longue d'environ une heure, soit de l'ordre de 5 à 6 heures. La durée d'obtention reste ainsi bien inférieure que celles permises par d'autres techniques de l'art antérieur, tout en conservant de bonnes qualités d'innocuité et de biocompatibilité.

**<u>Nature du/des silanes</u>**

De préférence, au moins un silane ajouté dans l'étape b), ou l'étape e), ou les deux, est un silane du type :

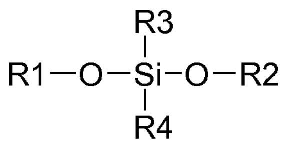

dans lequel les quatre groupements R1, R2, R3 et R4 peuvent être différents les uns des autres ou identiques entre eux, en tout ou partie.

Dans ce silane, ces quatre groupements R1, R2, R3 et R4 sont choisis parmi : alkyl, aryl, alkylaryl, alkylamine, hydroxyl, éther, ester, acide, Cl, Br, I, F, ou un groupement ethoxy possiblement hydrolisé (pour R4), ou un groupement du type :

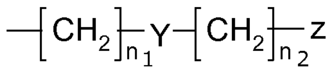

avec :

- n1 = 0 à 8 et n 2 = 0 à 10 ; et
- Y est choisi parmi : O, NH, S, Si, NR' ; et
- Z est choisi parmi : $CH_3$, $NH_2$, SH, Cl, Br, I, Cl, groupement glycosidique, hydroxyl, acide, ether,ester, amide, NH-R', NR'-R"

et dans lesquels (pour Y et Z) :

  - R' est choisi parmi alkyl, aryl, alkylaryl, alkylamine, éther, ester, cétone, cycle ramifié, et
  - R" est choisi parmi : alkyl, aryl, alkylaryl, alkylamine

**<u>Exemples de silanes</u>**

A titre d'exemple, un ou plusieurs des silanes utilisés dans l'étape b), ou l'étape e), ou les deux, peut être choisi parmi les corps suivants :

(3-(Trimethoxysilyl)propyl)diethylenetriamine, (3-chloropropyl)triethoxysilane, 1-[3-(trimethoxysilyl)]-propylurea, 3-[2-(2-Aminoethylamino)ethylamino]propyl-trimethoxysilane, 3-aminopropyl-diethoxy-methylsilane, 3-Aminopropylméthyldiéthoxysilane, 3-aminopropyltriéthoxysilane, 3-Aminopropyltriméthoxysilane, 3-Glycidyloxypropyltriéthoxysilane, 3-Mercaptopropyltriméthoxysilane, 3-Méthacryloxypropyltriméthoxysilane, Aminopropylméthyldiéthoxysilane, Bis(3-triéthoxysilylpropyl)amine, diethoxydimethylsilane, methyltriethoxysilane (MTES), Méthyltriméthoxysilane, N-(2-Aminoéthyl)-3-aminopropyltriméthoxysilane, Phényltriéthoxysilane, Propyltriméthoxysilane, Tétraéthoxysilane, tetraethylorthosilicate (TEOS), Tétraméthylorthosilicate, triethoxy(octyl)silane, Tris[3-(trimethoxysilyl)-propyl]isocyanurate, Vinyltriéthoxysilane, Vinyltriméthoxysilane.

Le tableau suivant présente plusieurs combinaisons de choix de silanes qui ont été expérimentées par les

inventeurs :

| étape b) | | | étape e) |
|---|---|---|---|
| TEOS | tétraéthoxy(octyl)silane | | |
| TEOS | MTES | | |
| TEOS | 3-aminopropyl diethoxymethylsilane | | |
| TEOS | MTES | 3-aminopropyl diethoxymethylsilane | |
| TEOS | phenyltriethoxysilane | | |
| TEOS | 1-[3-(trimethoxysylil)-propyl]urée | | |
| TEOS | triethoxyoctylsilane | | |
| TEOS | diethoxydimethylsilane | | |
| TEOS | 3-aminopropyltriethoxysilane | | |
| TEOS | tris(3-trimethoxysilyl-propyl)isocyanate | | |
| TEOS | phenyltriethoxysilane | diethoxydimethylsilane | |
| TEOS | phenyltriethoxysilane | diethoxydimethylsilane | 3-aminopropyl triethoxysilane |
| TEOS | diethoxydimethylsilane | | 3-aminopropyl diethoxymethylsilane |
| TEOS | diethoxydimethylsilane | | |
| TEOS | 3-aminopropyldiethoxymethylsilane | | |
| TEOS | diethoxydimethylsilane | | tris(3-trimethoxysilyl-propyl)isocyanate |
| TEOS | diethoxydimethylsilane | | triethoxyoctylsilane |
| TEOS | diethoxydimethylsilane | | phenyltriethoxy silane |
| TEOS | diethoxydimethylsilane | | 1-[3-(trimethoxysylil)-propyl]urée |
| TEOS | MTES | | 3-aminopropyl diethoxymethylsilane |
| TEOS | diethoxydimethylsilane | | 3-[2-(2-aminoethyl amino)ethylamino] propyl -trimethoxysilane |
| TEOS | diethoxydimethylsilane | (3-chloropropyl) triethoxysilane) | |
| TEOS | diethoxydimethylsilane | triethoxy(octyl)silane | |

De préférence, mais non obligatoirement, l'étape b) comprend l'ajout d'au moins deux silanes, par exemple d'une part de TEOS et d'autre part d'au moins un deuxième autre silane.

De préférence, l'étape c) est interrompue alors qu'il subsiste encore des groupes silanol.

En outre, selon une particularité optionnelle de l'invention, au moins un silane ajouté dans l'étape e) est un silane fonctionnalisé comprenant par exemple une fonction amine ou chlorée. La présence de groupements fonctionnalisants, par exemple $NH_2$, fournit des précurseurs à la formation de liaisons covalentes pouvant se fixer à d'autres composés, choisis en fonction de caractéristiques supplémentaires d'association ou de fixation qui sont recherchées pour les microcapsules produites, par exemple pour obtenir une liaison avec les fonctions hydroxyl présentes dans la cellulose du coton et ainsi obtenir une fixation des capsules au tissu.

## Préparation des microcapsules

L'invention propose la préparation de microcapsules contenant des composés pouvant présenter des propriétés lipophiles ou hydrophiles dont la double membrane en polysiloxane leur permet d'être plus étanches que dans l'état de

la technique. Cette membrane est assez étanche pour être utilisée pour encapsuler des composés volatils. Les développements de l'invention comprennent aussi la mise au point d'une méthode pour pouvoir comparer l'étanchéité des microcapsules entre différents procédés de préparation.

Des microcapsules ont été fabriquées selon l'invention, contenant une préparation à base d'une phase huileuse, une huile de tournesol, contenant un composé volatil, le limonène, et un composé non volatil, le salicylate de benzyle. Le procédé selon l'invention a été développé à partir du procédé d'encapsulation décrit dans le brevet EP 2 080 552, et le protocole de fabrication n'est décrit ici que dans ses différences.

Cette encapsulation selon l'invention utilise une phase aqueuse acide, avec un pH entre 2,5 et 4,5, et par exemple de 3, obtenu par un ou plusieurs acides faibles, et de préférence un mélange d'acide acétique et d'acide formique, et qui est chauffée entre 40°C et 70°C, et de préférence à 50°C.

A cette phase aqueuse on ajoute des tensio-actifs, dont au moins un cationique, par exemple des dérivés de cellulose comme par exemple un dérivé cationique de l'hydroxyéthylcellulose. On ajoute ensuite une phase huileuse, composée d'huile de tournesol, de salicylate de benzyle et de limonène.

Sous forte agitation, une émulsion huile dans eau est formée comprenant des gouttelettes huileuses renfermant les produits à encapsuler.

On ajoute alors du tetraethoxysilane (TEOS) et un autre silane, par exemple du dimethyldiethoxysilane, du phenyltriethoxysilane, ou du methyltriethoxysilane (MTES). La présence d'acide va hydrolyser les silanes qui vont former des silanols et migrer à l'interface eau/huile autour des gouttelettes, avec par exemple les réactions :

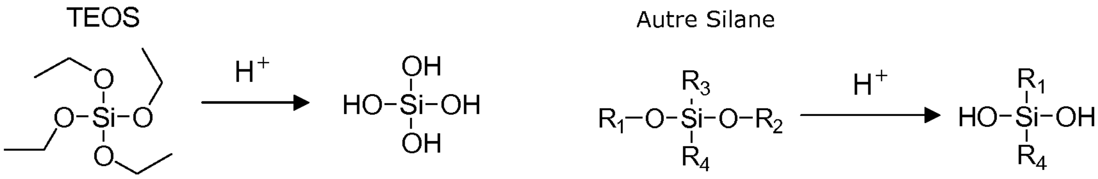


On effectue alors une remontée du pH à l'aide d'une base jusqu'à une valeur située entre 4,5 et 6 et de préférence 5,5, par exemple de l'ammoniaque ou de la soude, ou du di-éthanol-amine, ou de l'éthanolamine. Cette remontée du pH va permettre le début de la condensation et former ainsi une première membrane autour des gouttelettes renfermant le produit (ou les produits) à encapsuler. On ajuste la durée de cette condensation pour que des groupes silanol (Si-OH) restent présents à la surface de la membrane formée, à une valeur située entre 20 et 40 minutes, et de préférence environ 30 minutes.

On obtient ainsi une condensation partielle et une formation d'une membrane, avec par exemple la réaction suivante avec un silane trihydroxylé :

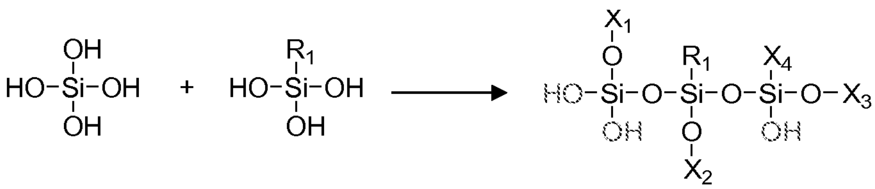

avec :

- $X_1$, $X_2$, $X_3$ = chaînes polymériques silylées ou H ; et
- $X_4$ = chaînes polymériques silylées, H ou $R_1$.

Le pH est alors ensuite redescendu entre 2,5 et 4,5, et de préférence aux alentours de 3,80, pendant une durée d'au moins 5 minutes, et de préférence environ 10 minutes, par exemple à l'aide d'un ou plusieurs acides faibles, et de préférence un mélange d'acide formique et d'acide acétique. Cette acidification stoppe la condensation et crée les conditions pour une nouvelle hydrolyse.

Dans un premier mode de réalisation, on ajoute alors un silane. Optionnellement, il peut s'agir d'un silane préhydrolysé, ce qui permet de compléter l'action d'hydrolyse du milieu acide. Cela permet aussi d'obtenir des groupements $NH_3^+$ en plus des groupements hydroxyl dans le cas d'une fonctionnalisation par groupements amine. Dans le cas où l'on recherche une membrane fonctionnalisée, on choisit un silane fonctionnalisé parmi ceux-ci indiqués précédemment, par exemple un aminosilane ou un silane chloré, dont les groupements fonctionnalisant (par exemple $NH_2$) vont favoriser la formation ultérieure de liaisons covalentes.

Par exemple, des expérimentations ont donné de bons résultats réalisées avec un silane non fonctionnalisé

pour l'étape b) (pour la première membrane) et un silane fonctionnalisé pour l'étape e) (pour la deuxième membrane).

A titre de d'exemple, la préhydrolyse de l'aminosilane comprend la réaction :

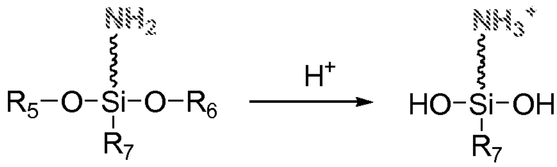

Avec R5, R6 et R7 décrits comme précédemment pour R1, R2 et R3.

Remarque : R7, qui peut être un groupement éther, peut également être hydrolysé.

Dans un deuxième mode de réalisation donnant lui aussi de bons résultats d'étanchéité, on effectue l'étape e) de remontée du pH sans ajouter de silane ou en ajoutant le même silane que dans l'étape b)

Dans les deux modes de réalisation, le pH est ensuite remonté à une valeur comprise entre 4,5 et 7, et de préférence environ 5,5, à l'aide d'une base, par exemple de l'ammoniaque ou de la soude, ou du di-éthanol-amine, ou de l'éthanolamine. La condensation se réalise entre les groupements silanol encore présents de la première membrane et ceux du silane (ou aminosilane) possiblement rajouté, avec par exemple la réaction suivante :

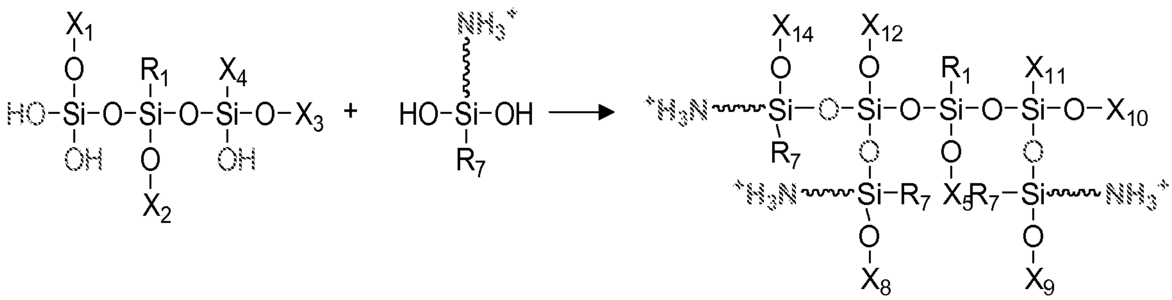

Avec :

- $X_5$ à $X_{14}$ = chaine polymérique silylée ou H ;
- Ou $X_{11}$ = chaine polymérique silylée, H ou $R_1$.

Si R7 est hydrolysé, le groupement hydroxyl obtenu peut alors également donner lieu à une chaine polymérique lors de la condensation, ce qui donne des possibilités supplémentaires de choix de structure.

Une membrane étanche est ainsi formée autour des gouttelettes contenant le ou les produit(s) à encapsuler, qui est aussi fonctionnalisée dans le cas d'un aminosilane.

Au final, on obtient un « slurry », c'est-à-dire une suspension de microcapsules dans une phase aqueuse. Cette suspension peut par exemple être transformée selon des procédés connus pour fournir une poudre de microcapsules, forme pour laquelle le procédé selon l'invention est particulièrement performant.

## <u>Essais d'étanchéité</u>

Les microcapsules préparées comme décrits ci-dessus ont alors été testées pour vérifier leur étanchéité, selon la méthode suivante.

Ces microcapsules préparées contiennent, au sein de leur phase d'huile de tournesol :

- un composé chimique pur lipophile et volatil : du (R)-limonène, et
- un composé chimique pur lipophile et non volatil : du salicylate de benzyle.

Pour évaluer la porosité des microcapsules, des échantillons de slurries sont mis à chauffer à différentes températures et pendant différentes durées, et un dosage par GCMS basé sur les aires des pics du limonène et du salicylate de benzyle est fait pour connaître les quantités de ces deux corps présentes dans les capsules.

A un instant $T_0$, pour un lot déterminé de microcapsules, on mesure le ratio des quantités de composés volatil et non volatil pour obtenir le ratio :

$$X = \frac{\text{Quantité de volatil}}{\text{Quantité de non volatil}}$$

Ce lot de microcapsules est passé au four, à une température par exemple de 80°C et/ou 120°C ou entre les deux, voire jusqu'à 160°C.

Après passage au four, on mesure à nouveau le ratio des quantités de composés volatil et non volatil, pour obtenir le ratio :

$$Y = \frac{\text{Quantité de volatil}}{\text{Quantité de non volatil}}$$

Le rapport entre les ratios X et Y, c'est à dire avant et après passage au four, donne une valeur indiquant la porosité des capsules.

Un rapport Y/X=100% indique que les capsules sont bien étanches. Une valeur plus petite que 100% indique qu'une partie des composés volatils s'est échappée, et donc que les capsules sont poreuses. Plus les capsules sont poreuses, plus le rapport Y/X sera petit.

Les rapports Y/X sont déterminés pour différents temps T de chauffage pour différentes microcapsules.

En reportant Y/X = f(T) pour chaque slurry, on obtient des courbes comparatives de la porosité.

Il est ainsi possible d'évaluer l'étanchéité des capsules obtenues par différents protocoles.

La FIGURE 1 illustre ainsi des résultats d'essais de porosité comparée, selon la durée de chauffage, entre :

- d'une part des capsules à membrane simple obtenues selon l'art antérieur tel que décrit dans le document EP 2 080 552, représentées par la courbe descendante avec losanges, et
- d'autre part des capsules à membrane double réalisées selon l'invention avec un aminosilane, représentées par la courbe stable avec carrés.

Cette courbe est tracée à partir des résultats suivants :

| durée | membrane simple selon l'art antérieur | membrane double selon l'invention |
|---|---|---|
| 0 min | 100% | 100% |
| 30 min | 90% | 97% |
| 60 min | 51% | 95% |
| 90 min | 45% | 96% |

On voit que la membrane double selon l'invention permet d'obtenir une bien meilleure étanchéité, en particulier à 60 min et 90 min.

De la même façon, le tableau suivant présente les résultats des mêmes essais de porosité comparée réalisés pour des microcapsules à membrane simple selon l'invention, tel que décrit ci-dessus pour le deuxième mode de réalisation :

| durée | membrane simple selon l'invention - essai 1 | membrane simple selon l'invention - essai 2 |
|---|---|---|
| 0 min | 100% | 100% |
| 30 min | 88% | 92% |
| 60 min | 79% | 99% |
| 90 min | 84% | 96% |

Par rapport aux microcapsules à membrane simple selon l'art antérieur (résultats dans le tableau précédent), on voit que le procédé selon l'invention permet une amélioration non négligeable même dans le mode de réalisation à membrane simple.

On voit ainsi que le procédé selon l'invention permet d'obtenir une réelle étanchéité, même à des durées où

les capsules selon l'art antérieur perdent 55% des composés volatils qu'elles contiennent. Cela représente un avantage très important, par exemple pour améliorer la durée de vie ou de péremption de nombreux produits tels que par exemple des « texticaments » ou des textiles à composante cosmétique, et ce dans des situations environnementales variées.

Cette technique a ainsi permis de valider les améliorations d'étanchéité apportées par le procédé d'encapsulation selon l'invention.

Bien sûr, l'invention n'est pas limitée aux exemples qui viennent d'être décrits et de nombreux aménagements peuvent être apportés à ces exemples sans sortir du cadre de l'invention.

## Revendications

1. Procédé d'encapsulation d'un ou plusieurs produits pouvant présenter des propriétés lipophiles ou hydrophiles dans une membrane polysiloxane, comprenant les étapes suivantes :

   a)- formation de gouttelettes par une émulsion entre une phase huileuse contenant le produit à encapsuler et une phase aqueuse acide chauffée entre 40°C et 70°C, et en présence d'agents tensio-actifs ;
   b)- ajout et hydrolyse d'au moins un silane pour obtenir un silanol ;
   c)- augmentation du pH pour obtenir un début de condensation du silanol pour former une première membrane autour des gouttelettes du produit à encapsuler ;
   d)- baisse du pH ;
   e)- augmentation du pH pour obtenir une nouvelle ou meilleure condensation de silanol autour des gouttelettes du produit à encapsuler.

2. Procédé selon la revendication précédente, **caractérisé en ce que** l'étape e) comprend en outre un ajout d'au moins un silane.

3. Procédé selon l'une quelconque des revendications précédentes, **caractérisé en ce qu'**au moins un silane ajouté lors de l'étape e) est un silane différent du ou des silanes ajoutés lors de l'étape b), formant ainsi autour des gouttelettes du produit à encapsuler une deuxième membrane lors de la nouvelle condensation de silanol au cours de l'étape e).

4. Procédé selon l'une quelconque des revendications précédentes, **caractérisé en ce qu'**au moins un silane ajouté dans l'étape b), ou l'étape e), ou les deux, est un silane du type :

   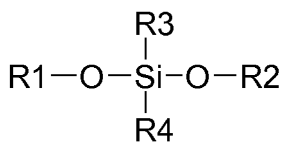

   dans lequel les quatre groupements R1, R2, R3 et R4 peuvent être différents les uns des autres ou identiques entre eux, en tout ou partie ;
   et dans lequel, lesdits groupements R1, R2, R3 et R4 sont choisis parmi : alkyl, aryl, alkylaryl, alkylamine, hydroxyl, éther, ester, acide, Cl, Br, I, F, ou un groupement ethoxy possiblement hydrolisé pour R4, ou un groupement du type :

   $$-\left[CH_2\right]_{n_1}-Y-\left[CH_2\right]_{n_2}-Z$$

   avec :

   - $n_1$ = 0 à 8 et $n_2$ = 0 à 10 ; et
   - Y est choisi parmi : O, NH, S, Si, NR' ; et
   - Z est choisi parmi : $CH_3$, $NH_2$, SH, Cl, Br, I, Cl, groupement glycosidique, hydroxyl, acide, ether,ester, amide, NH-R', NR'-R"

   et dans lesquels :

- R' est choisi parmi alkyl, aryl, alkylaryl, alkylamine, ether, ester, cétone, cycle ramifié, et
- R" est choisi parmi : alkyl, aryl, alkylaryl, alkylamine.

5. Procédé selon l'une quelconque des revendications précédentes, **caractérisé en ce qu'**un ou plusieurs des silanes utilisés dans l'étape b), ou l'étape e), ou les deux, est choisi parmi les corps suivants :

   (3-(Trimethoxysilyl)propyl)diethylenetriamine, (3-chloropropyl)triethoxysilane, 1-[3-(trimethoxysilyl)]-propylurea, 3-[2-(2-Aminoethylamino)ethylamino]propyl-trimethoxysilane, 3-aminopropyl-diethoxy-methylsilane, 3-Aminopropylméthyldiéthoxysilane, 3-aminopropyltriéthoxysilane, 3-Aminopropyltriméthoxysilane, 3-Glycidyloxypropyltriéthoxysilane, 3-Mercaptopropyltriméthoxysilane, 3-Méthacryloxypropyltriméthoxysilane, Aminopropylméthyldiéthoxysilane, Bis(3-triéthoxysilylpropyl)amine, diethoxydimethylsilane, methyltriethoxysilane (MTES), Méthyltriméthoxysilane, N-(2-Aminoéthyl)-3-aminopropyltriméthoxysilane, Phényltriéthoxysilane, Propyltriméthoxysilane, Tétraéthoxysilane, tetraethylorthosilicate (TEOS), Tétraméthylorthosilicate, triethoxy(octyl)silane, Tris[3-(trimethoxysilyl)-propyl]isocyanurate, Vinyltriéthoxysilane, Vinyltriméthoxysilane.

6. Procédé selon l'une quelconque des revendications précédentes, **caractérisé en ce que** l'étape b) comprend l'ajout de TEOS et d'au moins un deuxième silane.

7. Procédé selon l'une quelconque des revendications précédentes, **caractérisé en ce que** dans l'étape e) au moins un silane ajouté est un silane fonctionnalisé comprenant une fonction amine ou chlorée.

8. Procédé selon l'une quelconque des revendications précédentes, **caractérisé en ce que** le silane ajouté à l'étape e) est préhydrolysé.

9. Procédé selon l'une quelconque des revendications précédentes, **caractérisé en ce que** l'étape a) comprend les sous étapes suivantes :

   a1)- chauffage entre 40°C et 70°C et de préférence à 50°C d'une phase aqueuse acide ;
   a2)- ajout d'agent tensio-actif ;
   a3)- ajout d'une phase huileuse comprenant le produit à encapsuler ;
   a4)- agitation pour former une émulsion huile dans eau.

10. Procédé selon l'une quelconque des revendications précédentes, **caractérisé en ce que** dans l'étape a) la phase aqueuse présente un pH entre 2,5 et 4,5, et notamment de 3.

11. Procédé selon l'une quelconque des revendications précédentes, **caractérisé en ce que** dans l'étape c), le pH est augmenté de 2 à 3 jusqu'à une valeur allant de 4,5 à 6 et notamment de 5,5.

12. Procédé selon l'une quelconque des revendications précédentes, **caractérisé en ce que** l'étape c) est interrompue alors qu'il subsiste encore des groupes silanol.

13. Procédé selon l'une quelconque des revendications précédentes, **caractérisé en ce que** dans l'étape d), le pH est descendu jusqu'à une valeur allant de 2,5 à 4,5, et notamment à 3,8.

14. Procédé selon l'une quelconque des revendications précédentes, **caractérisé en ce que** dans l'étape e), le pH est augmenté jusqu'à une valeur allant de 4,5 à 7, et notamment de 5,5.

15. Procédé selon l'une quelconque des revendications précédentes, **caractérisé en ce que** dans l'étape a) ou dans l'étape d) ou les deux l'acidification est obtenue à l'aide d'acide acétique ou d'acide formique ou des deux.

16. Procédé selon l'une quelconque des revendications précédentes, **caractérisé en ce que** dans l'étape c) ou l'étape e) le pH est augmenté par ajout d'au moins une base, par exemple de l'ammoniaque, ou de la soude, ou du diéthanol-amine, ou de l'éthanolamine.

17. Procédé selon l'une quelconque des revendications précédentes, **caractérisé en ce que** les tensio-actifs comprennent un dérivé de cellulose.

## Patentansprüche

1. Verfahren zur Einkapselung eines oder mehrerer Produkte, die lipophile oder hydrophile Eigenschaften aufweisen können, in einer Polysiloxan-Membran, umfassend die folgenden Schritte:

   a)- Bilden von Tröpfchen durch eine Emulsion zwischen einer Ölphase, die das einzukapselnde Produkt enthält, und einer zwischen 40 °C und 70 °C erhitzten wässrigen sauren Phase, und in Gegenwart von oberflächenaktiven Substanzen;
   b)- Zugabe und Hydrolyse von wenigstens einem Silan, um ein Silanol zu erhalten;
   c)- Erhöhen des pH-Wertes, um einen Anfang einer Kondensation des Silanols zu erreichen, um eine erste Membran um die Tröpfchen des einzukapselnden Produkts zu bilden;
   d)- Senken des pH-Wertes;
   e)- Erhöhen des pH-Wertes, um eine erneute oder bessere Silanol-Kondensation um die Tröpfchen des einzukapselnden Produkts zu erreichen.

2. Verfahren nach dem vorhergehenden Anspruch, **dadurch gekennzeichnet, dass** Schritt e) ferner ein Zugeben wenigstens eines Silans umfasst.

3. Verfahren nach einem der vorhergehenden Ansprüche, **dadurch gekennzeichnet, dass** wenigstens ein während des Schrittes e) zugegebenes Silan ein Silan ist, das von dem oder den während des Schrittes b) zugegebenen Silanen verschieden ist, wodurch um die Tröpfchen des einzukapselnden Produkts eine zweite Membran während der erneuten Silanol-Kondensation im Laufe des Schrittes e) gebildet wird.

4. Verfahren nach einem der vorhergehenden Ansprüche, **dadurch gekennzeichnet, dass** wenigstens ein bei Schritt b), oder Schritt e), oder beiden, zugegebenes Silan ein Silan vom Typ

   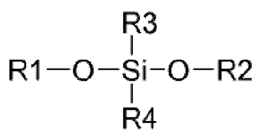

   ist, bei dem die vier Gruppen R1, R2, R3 und R4 in Gänze oder teilweise voneinander verschieden oder untereinander identisch sein können;
   und bei dem die Gruppen R1, R2, R3 und R4 ausgewählt sind aus: Alkyl, Aryl, Alkylaryl, Alkylamin, Hydroxyl, Ether, Ester, Säure, Cl, Br, I, F, oder einer möglicherweise hydrolysierten Ethoxygruppe für R4 oder einer Gruppe vom Typ:

   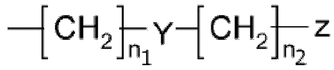

   mit:

   • $n_1$ = 0 bis 8 und $n_2$ = 0 bis 10; und
   • Y ist ausgewählt aus: O, NH, S, Si, NR'; und
   • Z ist ausgewählt aus: $CH_3$, $NH_2$, SH, Cl, Br, I, Cl, Glykosidgruppe, Hydroxyl, Säure, Ether, Ester, Amid, NH-R', NR'-R",

   und bei denen:

   • R' ausgewählt ist aus Alkyl, Aryl, Alkylaryl, Alkylamin, Ether, Ester, Keton, verzweigter Ring, und
   • R" ausgewählt ist aus: Alkyl, Aryl, Alkylaryl, Alkylamin.

5. Verfahren nach einem der vorhergehenden Ansprüche, **dadurch gekennzeichnet, dass** eines oder mehrere der bei Schritt b), oder Schritt e), oder beiden, verwendeten Silane aus den folgenden Körpern ausgewählt ist:

   (3-(Trimethoxysilyl)propyl)diethylentriamin, (3-Chlorpropyl)triethoxysilan, 1-[3-(Trimethoxysilyl)]-propylurea,

3-[2-(2-Aminoethylamino)ethylamino]propyl-trimethoxysilan, 3-Aminopropyl-diethoxy-methylsilan, 3-Aminopropylmethyldiethoxysilan, 3-Aminopropyltriethoxysilan, 3-Aminopropyltrimethoxysilan, 3-Glycidyloxypropyltriethoxysilan, 3-Mercaptopropyltrimethoxysilan, 3-Methacryloxypropyltrimethoxysilan, Aminopropylmethyldiethoxysilan, Bis(3-triethoxysilylpropyl)amin, Diethoxydimethylsilan, Methyltriethoxysilan (MTES), Methyltrimethoxysilan, N-(2-Aminoethyl)-3-aminopropyltrimethoxysilan, Phenyltriethoxysilan, Propyltrimethoxysilan, Tetraethoxysilan, Tetraethylorthosilikat (TEOS), Tetramethylorthosilikat, Triethoxy(octyl)silan, Tris[3-(trimethoxysilyl)-propyl]isocyanurat, Vinyltriethoxysilan, Vinyltrimethoxysilan.

**6.** Verfahren nach einem der vorhergehenden Ansprüche, **dadurch gekennzeichnet, dass** Schritt b) die Zugabe von TEOS und von wenigstens einem zweiten Silan umfasst.

**7.** Verfahren nach einem der vorhergehenden Ansprüche, **dadurch gekennzeichnet, dass** bei Schritt e) wenigstens ein zugegebenes Silan ein funktionalisiertes Silan mit einer Amin- oder Chlor-Funktion ist.

**8.** Verfahren nach einem der vorhergehenden Ansprüche, **dadurch gekennzeichnet, dass** das bei Schritt e) zugegebene Silan vorhydrolysiert ist.

**9.** Verfahren nach einem der vorhergehenden Ansprüche, **dadurch gekennzeichnet, dass** Schritt a) die folgenden Teilschritte umfasst:

a1)- Erhitzen einer wässrigen sauren Phase zwischen 40 °C und 70 °C und vorzugsweise auf 50 °C;
a2)- Zugeben einer oberflächenaktiven Substanz;
a3)- Zugeben einer Ölphase, die das einzukapselnde Produkt umfasst;
a4)- Rühren, um eine Öl-in-Wasser-Emulsion zu bilden.

**10.** Verfahren nach einem der vorhergehenden Ansprüche, **dadurch gekennzeichnet, dass** bei Schritt a) die wässrige Phase einen pH-Wert zwischen 2,5 und 4,5 und insbesondere von 3 aufweist.

**11.** Verfahren nach einem der vorhergehenden Ansprüche, **dadurch gekennzeichnet, dass** bei Schritt c) der pH-Wert um 2 bis 3 bis auf einen Wert von 4,5 bis 6 und insbesondere von 5,5 erhöht wird.

**12.** Verfahren nach einem der vorhergehenden Ansprüche, **dadurch gekennzeichnet, dass** Schritt c) unterbrochen wird, während weiterhin Silanol-Gruppen vorhanden sind.

**13.** Verfahren nach einem der vorhergehenden Ansprüche, **dadurch gekennzeichnet, dass** bei Schritt d) der pH-Wert bis auf einen Wert von 2,5 bis 4,5, und insbesondere auf 3,8, gesenkt wird.

**14.** Verfahren nach einem der vorhergehenden Ansprüche, **dadurch gekennzeichnet, dass** bei Schritt e) der pH-Wert bis auf einen Wert von 4,5 bis 7 und insbesondere von 5,5 erhöht wird.

**15.** Verfahren nach einem der vorhergehenden Ansprüche, **dadurch gekennzeichnet, dass** bei Schritt a) oder bei Schritt d) oder beiden die Ansäuerung mit Hilfe von Essigsäure oder Ameisensäure oder beiden erreicht wird.

**16.** Verfahren nach einem der vorhergehenden Ansprüche, **dadurch gekennzeichnet, dass** bei Schritt c) oder Schritt e) der pH-Wert durch Zugabe von wenigstens einer Base, beispielsweise Ammoniak in wässriger Lösung oder Soda oder Diethanolamin oder Ethanolamin erhöht wird.

**17.** Verfahren nach einem der vorhergehenden Ansprüche, **dadurch gekennzeichnet, dass** die oberflächenaktiven Substanzen ein Cellulosederivat umfassen.

## Claims

**1.** Method for encapsulating one or more products that can have lipophilic or hydrophilic properties in a polysiloxane membrane, comprising the following steps:

a) formation of droplets by an emulsion between an oily phase containing the product to be encapsulated and an acidic aqueous phase heated between 40°C and 70°C, and in the presence of surfactants;

b) addition and hydrolysis of at least one silane in order to obtain a silanol;
c) increasing the pH in order to start condensation of the silanol to form a first membrane around the droplets of the product to be encapsulated;
d) lowering the pH;
e) increasing the pH in order to obtain new or better condensation of the silanol around the droplets of the product to be encapsulated.

2. Method according to the preceding claim, **characterized in that** step e) further comprises the addition of at least one silane.

3. Method according to any one of the preceding claims, **characterized in that** at least one silane added during step e) is a silane different from the silane or silanes added during step b), thus forming a second membrane around the droplets of the product to be encapsulated during the new condensation of silanol during step e).

4. Method according to any one of the preceding claims, **characterized in that** at least one silane added in step b), or step e), or both, is a silane of the type:

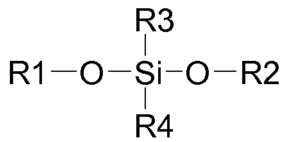

in which the four groups R1, R2, R3 and R4 can be different from one other or identical to one another, wholly or partly; and in which said groups R1, R2, R3 and R4 are selected from: alkyl, aryl, alkylaryl, alkylamine, hydroxyl, ether, ester, acid, Cl, Br, I, F, or an ethoxy group possibly hydrolysed for R4, or a group of the type:

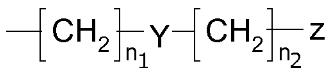

with:

- $n_1$ = 0 to 8 and $n_2$ = 0 to 10; and
- Y is selected from: O, NH, S, Si, NR'; and
- Z is selected from: $CH_3$, $NH_2$, SH, Cl, Br, I, Cl, glycosidic group, hydroxyl, acid, ether, ester, amide, NH-R', NR'-R"

and in which:

- R' is selected from alkyl, aryl, alkylaryl, alkylamine, ether, ester, ketone, branched ring, and
- R" is selected from: alkyl, aryl, alkylaryl, alkylamine.

5. Method according to any one of the preceding claims, **characterized in that** one or more silanes used in step b), or step e), or both, is selected from the following substances:

(3-(trimethoxysilyl)propyl)diethylenetriamine, (3-chloropropyl)triethoxysilane, 1-[3-(trimethoxysilyl)]-propylurea, 3-[2-(2-aminoethylamino)ethylamino]propyltrimethoxysilane, 3-aminopropyldiethoxymethylsilane, 3-aminopropylmethyldiethoxysilane, 3-aminopropyltriethoxysilane, 3-aminopropyltrimethoxysilane, 3-glycidyloxypropyltriethoxysilane, 3-mercaptopropyltrimethoxysilane, 3-methacryloxypropyltrimethoxysilane, aminopropylmethyldiethoxysilane, bis(3-triethoxysilylpropyl)amine, diethoxydimethylsilane, methyltriethoxysilane (MTES), methyltrimethoxysilane, N-(2-aminoethyl)-3-aminopropyltrimethoxysilane, phenyltriethoxysilane, propyltrimethoxysilane, tetraethoxysilane, tetraethylorthosilicate (TEOS), tetramethylorthosilicate, triethoxy(octyl)silane, tris[3-(trimethoxysilyl)-propyl]isocyanurate, vinyltriethoxysilane, vinyltrimethoxysilane.

6. Method according to any one of the preceding claims, **characterized in that** step b) comprises the addition of TEOS and of at least one second silane.

7. Method according to any one of the preceding claims, **characterized in that** in step e) at least one silane added is a functionalized silane comprising an amine or chlorinated function.

8. Method according to any one of the preceding claims, **characterized in that** the silane added in step e) is prehydrolysed.

9. Method according to any one of the preceding claims, **characterized in that** step a) comprises the following substeps:

a1) heating an acidic aqueous phase between 40°C and 70°C and preferably at 50°C;
a2) addition of surfactant;
a3) addition of an oily phase comprising the product to be encapsulated;
a4) stirring in order to form an oil-in-water emulsion.

10. Method according to any one of the preceding claims, **characterized in that** in step a) the aqueous phase has a pH between 2.5 and 4.5, and in particular of 3.

11. Method according to any one of the preceding claims, **characterized in that** in step c), the pH is increased from 2 to 3 up to a value in the range from 4.5 to 6 and in particular of 5.5.

12. Method according to any one of the preceding claims, **characterized in that** step c) is stopped while silanol groups still remain.

13. Method according to any one of the preceding claims, **characterized in that** in step d), the pH is lowered to a value in the range from 2.5 to 4.5, and in particular to 3.8.

14. Method according to any one of the preceding claims, **characterized in that** in step e), the pH is increased up to a value in the range from 4.5 to 7, and in particular of 5.5.

15. Method according to any one of the preceding claims, **characterized in that** in step a) or in step d) or both, acidification is obtained by means of acetic acid or formic acid or both.

16. Method according to any one of the preceding claims, **characterized in that** in step c) or step e) the pH is increased by adding at least one base, for example ammonia, or soda, or diethanolamine, or ethanolamine.

17. Method according to any one of the preceding claims, **characterized in that** the surfactants comprise a cellulose derivative.

Fig.1

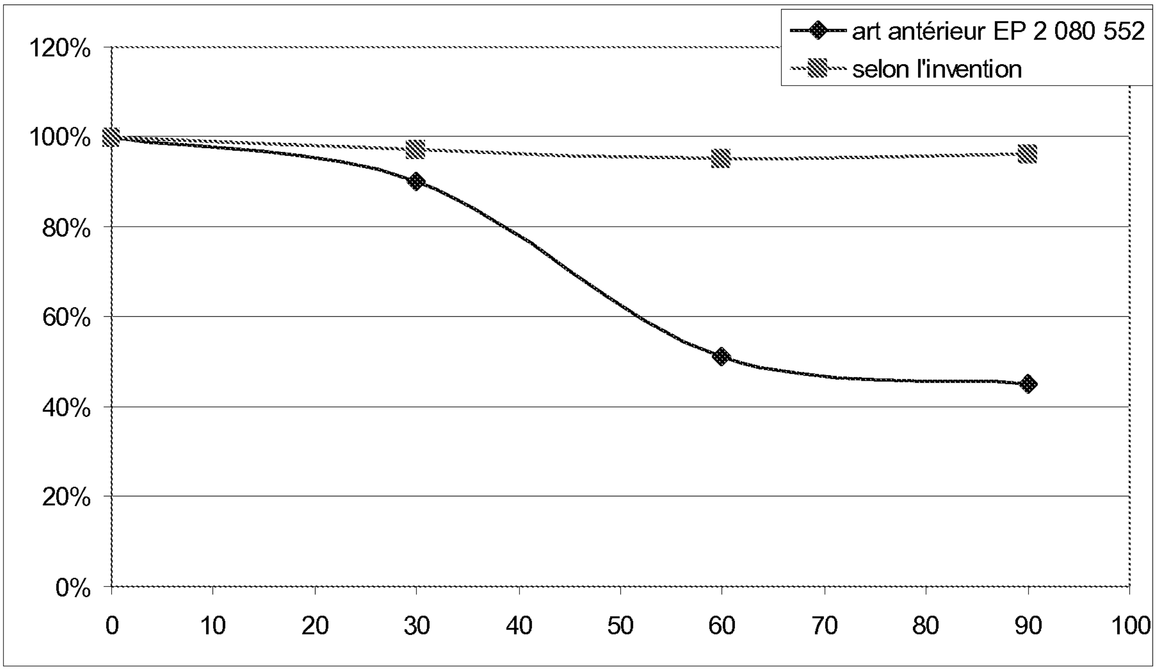
art antérieur EP 2 080 552
selon l'invention
120%
100%
80%
60%
40%
20%
0%
0
10
20
30
40
50
60
70
80
90
100

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6855335 B
- EP 2080552 A
- FR 2937248